(19) Europäisches Patentamt
European Patent Office
Office européen des brevets

(11) EP 4 768 028 A1

## (12) EUROPEAN PATENT APPLICATION

(43) Date of publication:
01.07.2026 Bulletin 2026/27

(51) International Patent Classification (IPC):
A61K 31/496 (2006.01)   A61P 25/16 (2006.01)
A61P 25/28 (2006.01)

(21) Application number: 25224862.0

(22) Date of filing: 18.12.2025

(52) Cooperative Patent Classification (CPC):
A61K 31/496; A61P 25/16; A61P 25/28

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 26.12.2024 KR 20240197149
16.09.2025 KR 20250132493

(71) Applicant: KNU-Industry Cooperation Foundation
Chuncheon-si, Gangwon-do 24341 (KR)

(72) Inventors:
• KIM, Jieun
24267 Chuncheon-si Gangwon-do (KR)
• CHAE, Sehyun
24267 Chuncheon-si Gangwon-do (KR)

(74) Representative: Isarpatent
Patent- und Rechtsanwälte PartG mbB
Friedrichstraße 31
80801 München (DE)

## (54) COMPOSITION FOR PREVENTING OR TREATING BRAIN-RELATED INFLAMMATION AND NEURODEGENERATIVE DISEASE COMPRISING ENTRECTINIB

(57) The present disclosure relates to a composition for preventing or treating brain-related inflammation disease or neurodegenerative disease, comprising entrectinib. The composition exhibits an effect of inhibiting brain-related inflammation and alleviating memory impairment, and thus may be usefully used for the prevention, alleviation, or treatment of brain-related inflammation disease, neurodegenerative disease, memory impairment, etc.

EP 4 768 028 A1

**Description**

**CROSS-REFERENCE TO RELATED APPLICATIONS**

This application claims the priority of Korean Patent Application No. 10-2024-0197149 filed on December 26, 2024, and Korean Patent Application No. 10-2025-0132493 filed on September 16, 2025, in the Korean Intellectual Property Office, the disclosures of which are incorporated herein by reference.

**BACKGROUND**

**Field**

The present disclosure relates to a composition for preventing or treating brain-related inflammation and neurodegenerative disease including entrectinib.

**Description of the Related Art**

Brain-related inflammatory response is one representative pathological phenomenon shown in most neurodegenerative diseases such as Alzheimer's disease and Parkinson's disease, and neuronal cell death is promoted by inflammatory mediators such as inflammatory cytokines and reactive oxygen species (ROS) produced by immune cells according to the brain-related inflammatory response.

Alzheimer's disease is a neurodegenerative disease characterized by progressive memory loss and cognitive impairment, primarily in people over 65 years of age. At the pathological level, beta-amyloid protein deposits and neurofibrillary tau protein tangles are representative etiological factors. As Alzheimer's disease progresses, mental and behavioral symptoms such as personality changes, agitation, depression, delusions, hallucinations, increased aggression, and sleep disturbances are often accompanied in addition to cognitive decline. As the disease reaches the late stage, neurological disorders such as stiffness and gait abnormality, or physical complications such as urinary and fecal incontinence, infections, and bedsores are caused. When the brain tissue of Alzheimer's disease patients was examined under a microscope, characteristic lesions such as neuritic plaques and neurofibrillary tangles are observed, and when observed with the naked eye, overall brain atrophy is observed due to loss of nerve cells. These brain pathological findings are mainly limited to the hippocampus and the entorhinal cortex, which are major brain regions responsible for memory, in the early stages of the disease, but gradually spread to the entire brain through the parietal lobe, the frontal lobe, etc. As these brain pathological invasion regions progress, memory loss is mainly shown in the early stages, and then as the disease progresses, the clinical symptoms become more diverse and more severe with a gradual progression. However, a current treatment method for dementia is only treatment for alleviating symptoms rather than addressing the fundamental cause, so that there is a need to develop substances for inhibiting, delaying, or treating the onset of Alzheimer's disease.

Microglia is a type of immune cells and as macrophages of the brain, plays an important role in host defense and tissue repair in the central nervous system (CNS). Under normal conditions, glial cells (i.e., astrocytes and microglial cells) support and protect neurons from pathogens and also function to maintain synaptic homeostasis. The microglia are found primarily in the hippocampus and the cortex, which control learning and memory. The abnormal activation of microglia by external stimuli causes the initiation of inflammation, secretion of proinflammatory mediators, and a wide range of responses including neurotoxic factors and various cytokines, and is involved in neurodegenerative disorders including Alzheimer's disease, Parkinson's disease and stroke. Accordingly, the inhibition of abnormal activation of microglia may have valuable therapeutic potential for the treatment of inflammation-related diseases. BV2 microglia has been extensively used as a cellular model of neuroinflammation to investigate responses to proinflammatory cytokines and oxidative stress for screening of novel drug candidates.

Meanwhile, entrectinib is a targeted anticancer drug used to treat solid cancer expressed by NTRK gene fusion. The targeted anticancer drug refers to an anticancer drug that selectively inhibits the proliferation or survival of cancer cells by targeting and recognizing a specific part of the surface of cancer cells that are different from normal cells or a signaling system inside the cancer cells. Generally, in anticancer therapy, a therapeutic agent is determined according to an occurrence location of cancer, and entrectinib is a representative anticancer drug for all types of cancer treated by targeting NTRK gene mutations regardless of the occurrence location of cancer. The entrectinib is mainly used to treat a specific solid cancer and non-small cell lung cancer.

Therefore, the present inventors confirmed that entrectinib, as an anticancer agent for treating solid cancer or non-small cell lung cancer, had an effect of inhibiting brain-related inflammation, thereby confirming the usability as a therapeutic agent for diseases other than cancer, and then completed the present disclosure.

[Prior Arts]

[Patent Documents]

(Patent Document 0001) Korean Patent Registration No. 10-2718538

**SUMMARY**

An object of the present disclosure is to provide a pharmaceutical composition for preventing or treating brain-related inflammation disease or neurodegenerative disease, including entrectinib.

Further, another object of the present disclosure is to provide a food composition for preventing or alleviating brain-related inflammation disease or neurodegenerative disease, including entrectinib.

Further, yet another object of the present disclosure is to provide an animal feed composition for preventing or alleviating brain-related inflammation disease or neurodegenerative disease, including entrectinib.

Further, still another object of the present disclosure is to provide a pharmaceutical composition for preventing or treating memory impairment including entrectinib.

Further, still another object of the present disclosure is to provide a food composition for preventing or alleviating memory impairment including entrectinib.

Further, still another object of the present disclosure is to provide an animal feed composition for preventing or alleviating memory impairment including entrectinib.

Further, still another object of the present disclosure is to provide a method for reducing brain-related inflammation including administering a composition including entrectinib to a subject.

Further, still another object of the present disclosure is to provide a method for preventing or alleviating memory impairment including administering a composition including entrectinib to a subject.

Further, still another object of the present disclosure is to provide a method for increasing phagocytosis of microglia, including treating a composition including entrectinib *in vitro* or to a subject.

In order to achieve the object, an aspect of the present disclosure provides a pharmaceutical composition for preventing or treating brain-related inflammation disease or neurodegenerative disease, including entrectinib.

Further, another aspect of the present disclosure provides a food composition for preventing or alleviating brain-related inflammation disease or neurodegenerative disease, including entrectinib.

Further, yt another aspect of the present disclosure provides an animal feed composition for preventing or alleviating brain-related inflammation disease or neurodegenerative disease, including entrectinib.

The brain-related inflammation disease or neurodegenerative disease may be a patient group with increased tropomysin receptor kinase (TRK) activity.

The composition may decrease inflammatory cytokines and increase anti-inflammatory cytokines.

The composition may inhibit phosphorylation of JNK, p38, AKT, NF-KB or STAT3.

The composition may decrease the expression of CD16/32 and increase the expression of CD206.

The composition may reduce amyloid beta (A$\beta$) aggregates.

The composition may increase phagocytosis of microglia.

The composition may prevent or alleviate memory impairment caused by brain-related inflammation disease or neurodegenerative disease.

The neurodegenerative disease may be selected from the group consisting of Alzheimer's disease, Parkinson's disease, Pick's disease, Huntington's disease, dementia, mild cognitive impairment, stroke, and cerebral infarct.

Further, still another aspect of the present disclosure provides a pharmaceutical composition for preventing or treating memory impairment including entrectinib.

Further, still another aspect of the present disclosure provides a food composition for preventing or alleviating memory impairment including entrectinib.

Further, still another aspect of the present disclosure provides an animal feed composition for preventing or alleviating memory impairment including entrectinib.

The memory impairment may be caused by neuroinflammation.

Further, still another aspect of the present disclosure provides a method for reducing brain-related inflammation including administering a composition including entrectinib to a subject other than humans.

Further, still another aspect of the present disclosure provides a method for preventing or alleviating memory impairment including administering a composition including entrectinib to a subject other than humans.

Further, still another aspect of the present disclosure provides a method for increasing phagocytosis of microglia, including treating a composition including entrectinib *in vitro* or to a subject other than humans.

According to the present disclosure, the composition including entrectinib exhibits effects of inhibiting brain-related inflammation and alleviating memory impairment, and thus may be usefully used for preventing, alleviating, or treating brain-related inflammation disease, neurodegenerative disease, memory impairment, etc.

The effects of the present disclosure are not limited to the aforementioned effects, and other effects, which are not mentioned above, will be apparently understood to a person having ordinary skill in the art from the following description.

The objects to be achieved by the present disclosure, the means for achieving the objects, and the effects of the present disclosure described above do not specify essential features of the claims, and, thus, the scope of the claims is not limited to the disclosure of the present disclosure.

## BRIEF DESCRIPTION OF DRAWINGS

The above and other aspects, features and other advantages of the present disclosure will be more clearly understood from the following detailed description taken in conjunction with the accompanying drawings, in which:

FIG. 1 shows results of Western blot analysis for TRK phosphorylation after treatment with entrectinib in LPS-treated microglia (n = 4/group, *p < 0.05, **p < 0.01, ***p < 0.001, ****p < 0.0001);

FIG. 2 shows results of immunofluorescence analysis of p-TRK expression after treatment with entrectinib in LPS-treated microglia (n = 8/group, *p < 0.05, **p < 0.01, ***p < 0.001, ****p < 0.0001);

FIG. 3 shows results of real-time PCR analysis of proinflammatory factors and anti-inflammatory factors after treatment with entrectinib in LPS-treated microglia (n = 4/group, *p < 0.05, **p < 0.01, ***p < 0.001, ****p < 0.0001);

FIG. 4 shows results of Western blot analysis for JNK, P38 and AKT phosphorylation levels after treatment with entrectinib in LPS-treated microglia (JNK, n = 4-7/group; P38 and AKT, n = 4/group, *p < 0.05, **p < 0.01, ***p < 0.001, ****p < 0.0001);

FIG. 5 shows results of Western blot analysis for cytosolic and nuclear p-NF-KB and p-STAT3 levels after treatment with entrectinib in LPS-treated microglia (n = 3/group, *p < 0.05, **p < 0.01, ***p < 0.001, ****p < 0.0001);

FIG. 6 shows results of immunofluorescence analysis for CD16/32 and CD206 as reactive and protective microglia markers after treatment with entrectinib in LPS-treated microglia (n = 8/group, *p < 0.05, **p < 0.01, ***p < 0.001, ****p < 0.0001);

FIG. 7 shows results of live imaging analysis of phagocytosis in BV2 microglia treated with LPS, entrectinib, and Aβ oligomers (n = 8/group, *p < 0.05, **p < 0.01, ***p < 0.001, ****p < 0.0001);

FIG. 8 shows results of real-time PCR analysis for the expression of phagocytic factors and cytoskeletal genes in microglia treated with LPS and entrectinib (n = 3/group, *p < 0.05, **p < 0.01, ***p < 0.001, ****p < 0.0001);

FIG. 9 shows results of western blot analysis (n = 4/group) and immunofluorescence staining of TRK phosphorylation in the mouse hippocampus after administration with entrectinib and LPS (*p < 0.05, **p < 0.01, ***p < 0.001, ****p < 0.0001);

FIG. 10 shows results of western blot analysis for JNK, P38, and AKT phosphorylation in the mouse hippocampus after administration with entrectinib and LPS (n = 4/group, *p < 0.05, **p < 0.01, ***p < 0.001, ****p < 0.0001);

FIG. 11 shows results of immunofluorescence analysis for p-NF-KB and p-STAT3 in the microglial nuclei of the mouse hippocampus after administration with entrectinib and LPS (n = 3/group, *p < 0.05, **p < 0.01, ***p < 0.001, ****p < 0.0001);

FIG. 12 shows results of Y-maze and NOR tests in mice administered with entrectinib and LPS for 8 days (n = 6-8/group, *p < 0.05, **p < 0.01, ***p < 0.001, ****p < 0.0001);

FIG. 13 shows results of Y-maze and NOR tests in mice administered with entrectinib and LPS for 16 days (n = 6-8/group, *p < 0.05, **p < 0.01, ***p < 0.001, ****p < 0.0001);

FIG. 14 shows changes in body weight in mice administered with entrectinib and LPS for 8 or 16 days (n = 6-8/group, *p < 0.05, **p < 0.01, ***p < 0.001, ****p < 0.0001);

FIG. 15 shows results of analyzing synaptic change markers in mice administered with entrectinib and LPS for 8 days (n = 8/group, *p < 0.05, **p < 0.01, ***p < 0.001, ****p < 0.0001); and

FIG. 16 shows results of analyzing synaptic change markers in mice administered with entrectinib and LPS for 16 days (n = 8/group, *p < 0.05, **p < 0.01, ***p < 0.001, ****p < 0.0001).

## DETAILED DESCRIPTION OF THE EMBODIMENT

Hereinafter, aspects and exemplary embodiments of the present disclosure will be described in detail so as to be easily implemented by those skilled in the art, with reference to the accompanying drawings. However, the present disclosure may be implemented in various forms and is not limited to aspects and exemplary embodiments to be described herein.

Throughout the present specification, when a certain part "comprises" a certain component, unless otherwise disclosed to the contrary, it is meant that the part may further comprise another component without excluding another component.

The present disclosure provides a pharmaceutical composition for preventing or treating brain-related inflammation disease or neurodegenerative disease, including entrectinib.

In the present disclosure, "entrectinib" is a targeted anticancer agent used to treat solid cancer expressed by NTRK gene fusion, and is a representative anticancer agent for all cancer types treated by targeting NTRK gene mutations regardless of the occurrence location of cancer. The entrectinib is mainly used to treat specific solid cancer and non-small cell lung cancer.

The brain-related inflammation disease refers to an inflammatory disease of the brain parenchyma, and may be broadly classified into infectious, vasculitic, neoplastic, chemical, and idiopathic diseases depending on a cause, and includes Alzheimer's or Parkinson's disease, but is not limited thereto.

The neurodegenerative disease includes Alzheimer's disease, Parkinson's disease, Pick's disease, Huntington's disease, dementia, mild cognitive impairment, stroke, or cerebral infarct, but is not limited thereto, and preferably, may be neurodegenerative disease caused by brain-related inflammation.

The composition may prevent or alleviate memory impairment, and preferably prevent or alleviate memory impairment caused by brain-related inflammation disease or neurodegenerative disease, but is not limited thereto.

The brain-related inflammation disease or neurodegenerative disease may be a patient group with increased TRK activity, but is not limited thereto.

The composition may inhibit tropomysin receptor kinase (TRK) activity, preferably inhibit TRK activity increased by neuroinflammation, and more preferably inhibit TRK activity in microglia, but is not limited thereto.

The composition may decrease inflammatory cytokines and increase anti-inflammatory cytokines, preferably decrease or increase inflammatory cytokines and anti-inflammatory cytokines that are increased or decreased by neuroinflammation, and more preferably decrease or increase inflammatory cytokines and anti-inflammatory cytokines in microglia, but is not limited thereto.

The inflammatory cytokines include IL-1$\beta$, IL-6, TNF-$\alpha$, etc., and the anti-inflammatory cytokines include IL-4, IL-10, TGF-$\beta$, etc., but are not limited thereto.

The composition may inhibit phosphorylation of JNK, p38, AKT, NF-KB or STAT3, preferably inhibit phosphorylation of JNK, p38, AKT, NF-KB or STAT3 increased by neuroinflammation, and more preferably inhibit phosphorylation of JNK, p38, AKT, NF-KB or STAT3 in microglia, but is not limited thereto.

The composition may decrease the expression of CD16/32 and increase the expression of CD206, preferably decrease or increase the expression of CD16/32 and CD206 increased or decreased by neuroinflammation, and more preferably decrease or increase the expression of CD16/32 and CD206 in microglia, but is not limited thereto.

The composition may increase phagocytosis of microglia, and preferably increase phagocytosis of microglia decreased by neuroinflammation, but is not limited thereto.

The composition may reduce amyloid beta (A$\beta$) aggregates, and the reduction may be caused by activation of phagocytosis against amyloid beta aggregates, but is not limited thereto.

The pharmaceutical composition of the present disclosure may be formulated into powders, granules, tablets, coated tablets, pills, sugar-coated tablets, capsules, liquids, suspensions, gels, syrups, slurry agents, suppositories, emulsions, pastes, ointments, creams, lotions, powders, sprays or suspensions. The pharmaceutical composition may further include excipients, such as fillers, extenders, binders, wetting agents, disintegrants, surfactants, and diluents, which are commonly used for the formulation, and in addition to simple excipients, lubricants such as magnesium stearate and talc may also be used, but are not limited thereto.

The pharmaceutical composition may be administered parenterally or orally depending on an intended method, and the dose range varies depending on the weight, age, sex, health condition of a patient, diet, administration time, administration method, excretion rate, and severity of a disease. In addition, a therapeutically effective amount of the composition may vary depending on an administration method, a target site, and a patient's condition, and when used in the human body, the dose needs to be determined as an appropriate amount in consideration of both safety and effectiveness.

The pharmaceutical composition may further include an adjuvant in addition to the active ingredients. Any adjuvant known in the art may be used as the adjuvant without limitation.

Further, the present disclosure provides a food composition for preventing or alleviating brain-related inflammation disease or neurodegenerative disease, including entrectinib.

The food composition of the present disclosure may be health functional foods, dairy products, fermented products, or food additives. The health functional food refers to food produced and processed using raw materials or ingredients with functionality, which are useful for the human body, and the "functionality" means intake for adjusting nutrients for the structures and functions of the human body or obtaining a useful effect on health applications such as physiological actions.

The food composition includes formulations, such as pills, powders, granules, infusions, tablets, capsules, liquids, pastes, gels, or jellies, and the food composition of each formulation may be mixed with ingredients commonly used in the art, which are appropriately selected by those skilled in the art without difficulty according to a formulation or purpose of use, in addition to the active ingredients.

Further, the present disclosure provides an animal feed composition for preventing or alleviating brain-related inflammation disease or neurodegenerative disease, including entrectinib.

The animal feed composition of the present disclosure may be prepared separately in the form of feed additives to be mixed into the feed or prepared by directly adding the feed during the preparation of the feed, and may further include conventional additives capable of increasing the preservation of the feed.

The animal feed composition includes vegetable products, such as grains, root fruits, food processing by-products, algae, fibers, pharmaceutical by-products, oils and fats, starches, byproduct feed, grain by-products, etc.; and animal products, such as proteins, inorganic materials, fats and oils, minerals, single-celled proteins, zooplanktons, leftover food, etc., but is not limited thereto.

The animals are a biota corresponding to plants that mainly consume organic materials as nutrients and have differentiated digestive, excretory, and respiratory organs. Specifically, the animals may be echinoderms, crustaceans, mollusks, fish, amphibians, reptiles, birds, and mammals.

The animal feed composition may further include grains, vegetable protein feed, animal protein feed, sugar or dairy products, and may further use ingredients, such as nutritional supplements, digestion and absorption enhancers, growth promoters or disease preventive agents.

The feed composition may be prepared as a consistent coarse or granular material depending on the degree of grinding of ingredients, and the composition may be supplied as a mesh or formed into a desired separated shape for further processing and packaging, and may be pelletized, expanded or extruded for storage, and may preferably be dried to remove excess water for ease of storage.

Further, the present disclosure provides a pharmaceutical composition for preventing or treating memory impairment including entrectinib.

The memory impairment may be caused by neuroinflammation, preferably caused by LPS, but is not limited thereto.

The composition may increases synaptic proteins, and preferably increase synaptic proteins decreased by neuroinflammation, but is not limited thereto.

The synaptic proteins include synaptophysin (SYP), postsynaptic density protein 95 (PSD95), etc., but are not limited thereto.

Further, the present disclosure provides a food composition for preventing or alleviating memory impairment including entrectinib.

Further, the present disclosure provides an animal feed composition for preventing or alleviating memory impairment including entrectinib.

Further, the present disclosure provides a method for reducing brain-related inflammation including administering a composition including entrectinib to a subject.

The subject includes a subject in need of regulation or reduction of brain-related inflammation, and may preferably be a subject other than humans, but is not limited thereto.

Further, the present disclosure provides a method for preventing or alleviating memory impairment including administering a composition including entrectinib to a subject.

The subject includes a subject in need of prevention or alleviation of memory impairment, and may preferably be a subject other than humans, but is not limited thereto.

Further, the present disclosure provides a method for increasing phagocytosis of microglia including treating a composition including entrectinib *in vitro* or to a subject.

The subject includes a subject in need of regulation or increase in phagocytosis of microglia, and may preferably be a subject other than humans, but is not limited thereto.

As used in the present disclosure, the term "prevention" means all actions of inhibiting or delaying diseases, etc. by administering the composition according to the present disclosure. The "treatment" means all actions of improving or beneficially changing the symptoms of a suspected or developed subject by administering the composition. In addition, the "alleviation" means all actions that at least reduce parameters associated with conditions to be treated, for example, the degree of symptoms by administering the composition of the present disclosure.

In the present disclosure, '%' used to indicate a concentration of a specific material is solid/solid (w/w)%, solid/liquid (w/v)%, and liquid/liquid (v/v)%, unless otherwise stated.

Hereinafter, the present disclosure will be described in more detail through the following Examples, but the following Examples are only for illustrative purposes and are not intended to limit the scope of the present disclosure.

**[Preparation Example] Materials and Methods**

1. Treatment with entrectinib and LPS

In all *in vitro* experiments, lipopolysaccharide (LPS) from *Escherichia coli* (Sigma, Cat No. L2630, St. Louis, MO, USA) dissolved in PBS was used. Primary and BV2 microglia were treated with LPS at a concentration of 200 ng/mL. In the case of *in vivo* studies, mice were injected intraperitoneally (i.p.) with 250 $\mu$g/kg LPS once a day for 8 days to induce an inflammatory response.

Entrectinib (Selleckchem, Cat No. S7998, Houston, TX, USA) was applied in primary and BV2 microglia at a concentration of 1 $\mu$M unless otherwise stated to be prepared in dimethyl sulfoxide (DMSO) for *in vitro* treatment. In the case of *in vivo* experiments, a control solution consisted of 5% DMSO, 10% PEG, and 20% Tween 80 in deionized water. The mice were administered with entrectinib at a dose of 10 mg/kg.

## 2. BV2 microglia

A BV2 microglial cell line was purchased from Koram Bio Tech (Seoul, Korea).

The cells were incubated in a high-glucose Dulbecco's modified Eagle's medium (DMEM, Cytiva, Cat No. SH30243.01, Marlborough, MA, USA) and supplemented with 5% fetal bovine serum (FBS, Gibco, Cat No. 16000-044, Waltham, MA, USA), 100 U/mL penicillin G, and 100 $\mu$g/mL streptomycin. The incubation was maintained at 37°C in a 5% $CO_2$ incubator.

## 3. Incubation of primary microglia

Primary glial cells were isolated from the brains of C57BL/6J mice on postnatal day 1. The cerebra were added in cold HBSS, washed twice in low-glucose DMEM, filtered through a 70-$\mu$m nylon mesh, and incubated in low-glucose DMEM containing 10% FBS, 100 U/mL penicillin, and 100 $\mu$g/mL streptomycin in a 5% $CO_2$ incubator. On day 14, astrocytes were removed from the cells using 0.25% trypsin-EDTA in low glucose DMEM supplemented with EDTA and $CaCl_2$. After washing to remove the astrocyte layer, primary microglia were isolated with 0.25% trypsin-EDTA and centrifuged twice at 2000 rpm for 10 minutes before use.

## 4. CCK-8 assay

The cells were dispensed at a cell density of $1 \times 10^4$ cells per well in a 96-well plate and incubated in a FBS-free medium for 1 hour. Thereafter, the cells were treated with entrectinib at a concentration of 0.1, 1, 5, or 10 $\mu$M, or 1% DMSO as a control, for 12, 24, and 48 hours. At each time point, a 10% CCK-8 solution (Dojindo, Cat No. CK04, Kumamoto, Japan) was added to an FBS-free medium, and the cells were further incubated for 2 hours. Cell viability was confirmed by measuring absorbance at 450 nm using a Multiskan Skyhigh microplate spectrophotometer (Thermo Scientific, Waltham, MA, USA).

## 5. Real-time PCR

The mRNA levels of genes involved in inflammatory response *(Il6, Il1β, Tnfα, Inos, Ccl2, Il-23α, Il10, Il13, Il4)*, phagocytosis *(Trem2, Sorl1, Cd33, Cr2),* and cytoskeleton *(Vav1, Cdc42)* were evaluated using real-time PCR in primary microglia and hippocampal tissue. RNA was extracted from the primary microglia or hippocampal tissue using Nucleozol (Macherey-Nagel, Cat No. 740404.200, Dueren, Germany) according to the manufacturer's instructions. Complementary DNA (cDNA) was synthesized using Superscript cDNA Premix Kit II (GeNetBio, Cat No. SR-5000, Daejeon, Korea), and 40 cycles of real-time PCR were performed on a CFX Duet Real-Time PCR System (Bio-Rad, Hercules, CA, USA) using a SensiFAST™ SYBR No-ROX Kit (Bioline, Cat No. BIO-98050, Memphis, TN, USA). The *Gapdh* cycle threshold (Ct) was used for normalization. Fold changes in cells or tissues treated with LPS or LPS + entrectinib were calculated based on a control group.

## 6. Cytosolic and nuclear fractions

Nuclear levels of phosphorylated NF-KB (p-NF-KB) and phosphorylated STAT3 (p-STAT3) were evaluated in primary microglia treated with 200 ng/mL LPS or phosphate PBS for 30 minutes and then exposed to 1 $\mu$M entrectinib or 1% DMSO (control) for 5.5 hours. For cell lysis, the cells were suspended in a cytosolic fraction buffer consisting of 10 mM HEPES (pH 7.4), 10 mM KCl, and 0.05% NP-40 and incubated on ice for 20 minutes. Thereafter, the cells were centrifuged at 14,000 rpm for 10 minutes at 4°C. The supernatant representing the cytosolic fractionation was transferred to a new tube. Thereafter, a RIPA lysis buffer (Thermo Scientific, Cat No. 89901) was added to the remaining pellet and sonicated to lyse nuclear ingredients. The cells were further incubated on ice for 10 minutes, and then the nuclear lysates were centrifuged again at 14,000 rpm for 10 minutes at 4°C. The obtained nuclear fractions were used for Western blot analysis to detect nuclear p-NF-KB and p-STAT3 levels.

## 7. Western blot

Western blot was performed on primary microglia and hippocampal tissue treated with entrectinib or DMSO (control) after treated with LPS or PBS. Samples were lysed in an RIPA lysis buffer (Thermo Scientific, Cat No. 89901), isolated by ultrasound, and then centrifuged at 12,000 rpm for 10 minutes. The protein concentration in the supernatant was quantified compared with a standard BSA solution, and 20 $\mu$g of the proteins were loaded on an 8% or 10% SDS-PAGE gel depending on experimental requirements. The proteins were transferred to a nitrocellulose membrane (Amersham™ Protran™ 0.2 $\mu$m NC, Cytiva, Cat No. 10600001, Wilmington, DE, USA), blocked with 5% skim milk or 5% BSA for 1 hour at room temperature, and then incubated overnight with the following various antibodies: anti-p-TRK (pan) (1 : 1000, Cell Signaling Technology, Cat No. 4621, Danvers, MA, USA), anti-TRK (pan) (1 : 1000, Cell Signaling Technology, Cat No. 92991), anti-p-JNK (1 : 1000, Cell Signaling Technology, Cat No. 9251), anti-JNK (1 : 1000, MyBioSource, Cat No. MBS8509129, San Diego, CA, USA), anti-p-P38 (1 : 1000, Cell Signaling Technology, Cat No. 4511), anti-P38 (1 : 1000, Cell Signaling Technology, Cat No. 8690), anti-p-AKT (1 : 1000, Cell Signaling Technology, Cat No. 9271), anti-AKT (1 : 1000, Cell Signaling Technology, Cat No. 9272), anti-p-STAT3 (1 : 1000, Abcam, Cat No. ab32143, Cambridge, MA, USA), anti-STAT3 (1 : 1000, Abcam, Cat No. ab68153), anti-p-NF-KB (1 : 1000, Cell Signaling Technology, Cat No. 3033), anti-NF-KB (1 : 1000, Cell Signaling Technology, Cat No. 8242), anti-histone H3 (1 : 10,000, Cell Signaling Technology, Cat No. 9715), and anti-GAPDH (1 : 10,000 Cell Signaling Technology, Cat No. 2118) (4°C).

Thereafter, the membrane was incubated with HRP-conjugated goat anti-mouse IgG (1 : 10,000 Invitrogen, Cat No. 31430, Carlsbad, CA, USA) or anti-rabbit IgG (1 : 10,000 Promega, Cat No. W4011, Madison, WI, USA) antibody for 1 hour. The detection was performed using the ECL™ Prime Western Blotting System (Cytiva, Cat No. RPN2232). A Restore™ Western Blot Stripping Buffer (Thermo Scientific, Cat No. 21059) was used to detect additional proteins on the same membrane. Images were collected and analyzed using the ChemiDoc MP™ Imaging System (Bio-Rad, Hercules, CA, USA).

## 8. Immunocytochemistry

For immunocytochemistry, cells were fixed in 4% paraformaldehyde for 10 minutes and washed three times with PBS. Then, the cells were incubated for one day in PBS containing FBS using the following primary antibodies: anti-p-TRK (pan) (1 : 500, Cell Signaling Technology, Cat No. 4621), anti-p-JNK (1 : 100, Cell Signaling Technology, Cat No. 9251), anti-p-P38 (1 : 100, Cell Signaling Technology, Cat No. 4511), anti-p-AKT (1 : 1000, Cell Signaling Technology, Cat No. 9271), anti-p-STAT3 (1 : 100, Abcam, Cat No. ab32143), anti-p-NF-KB (1 : 100, Cell Signaling Technology, Cat No. 3033), anti-CD11b (1 : 100, Cell Signaling Technology, Cat No. 46512), anti-CD16/32 (1 : 100, Cell Signaling Technology, Cat No. 80366), and anti-CD206 (1 : 100, Cell Signaling Technology, Cat No. 24595). After incubation, the cells were washed with PBS and treated with Alexa Fluor 488- or 594-conjugated secondary antibodies for 2 hours at room temperature. The cells were washed three times again with PBS and incubated in a DAPI solution (Thermo Scientific, Cat No. 62248) for 10 minutes. After DAPI staining, the cells were washed and mounted on slides using a fluorescence-preserving solution (Dako, Cat No. S3023, Santa Clara, CA, USA), and images were taken with an A1/Ni-E microscope (Nikon, Tokyo, Japan). Image analysis was performed using ImageJ software.

## 9. Animal and immunofluorescence staining

Male C57BL6/J mice (8 weeks old; Orient-Bio Company, Gyeonggi-do, Korea) were maintained in a pathogen-free environment at 22 ± 2°C, 50 ± 5% humidity, and a 12-hour light/dark cycle, and freely fed feed and water. The mice were randomly divided into three groups of control, LPS, and LPS+entrectinib. Depending on the group, mice were administered with 10 mg/kg entrectinib or vehicle solution (5% DMSO, 10% PEG, 20% Tween 80 in deionized water) by intraperitoneal injection daily for 8 days. On day 8, 250 $\mu$g/kg LPS or PBS was administered intraperitoneally after 30 minutes of the final injection. After 8 hours of injection, the mice were perfused and fixed with PBS and 4% paraformaldehyde. The brain tissues were stored in 4% paraformaldehyde at 4°C for 24 hours, immersed in 30% sucrose of PBS for 72 hours, and then cut into 30 $\mu$m sections using a cryostat microtome (Leica CM1850, Wetzlar, Germany). The sections were blocked with 5% normal goat serum (Vector Laboratories, Burlingame, CA, USA) for 2 hours at room temperature and then immunostained with primary antibodies at 4°C for 1 day: anti-p-TRK (1 : 150, Cell Signaling Technology, Cat No. 4621), anti-CD11b (1 : 500, Cell Signaling Technology, Cat No. 46512), anti-p-NF-KB (1 : 500, Cell Signaling Technology, Cat No. 3033), anti-p-STAT3 (1 : 200, Abcam, Cat No. ab32143), anti-CD16/32 (1 : 200, Cell Signaling Technology, Cat No. 80366), and anti-CD206 (1 : 50, Cell Signaling Technology, Cat No. 24595). The sections were washed with a PBST buffer and incubated with Alexa 555- or Alexa 488-conjugated secondary antibodies for 2 hours at room temperature. The sections were washed three times again with a PBST buffer and incubated in a DAPI solution (Thermo Scientific, Cat No. 62248) for 10 minutes. After DAPI staining, fluorescence microscope images were captured using a DMi8 microscope (Leica Microsystems, Wetzlar, Germany) and analyzed with ImageJ software (US National Institutes of Health, Bethesda, MD, USA), after treatment with a VectaShield solution for fluorescence (Vector Labs, Cat No. H-1000, Burlingame, CA, USA).

## 10. Phagocytosis assay and live cell imaging

Phagocytosis of BV2 microglia was observed through A$\beta$ clearance. Before exposure to A$\beta$, BV2 microglia were treated with 200 ng/mL LPS or PBS for 30 minutes and then treated with 1 $\mu$M entrectinib or 1% DMSO (control) for 23.5 hours. During incubation, 250 nM Alexa Fluor 488-conjugated A$\beta_{1-42}$ (Anaspec, Cat No. AS-60479-01, Fremont, CA, USA) was incubated in a serum-free medium at 37°C for 24 hours. Then, BV2 microglia were exposed to pre-prepared A$\beta_{1-42}$ for 1 hour. Thereafter, the cells were fixed in 4% paraformaldehyde for 20 minutes, washed three times with PBS, and then incubated in a DAPI solution for 10 minutes. After DAPI staining, the cells were washed and mounted on slide glass using a fluorescent solution (Dako, Cat No. S302380-2, Santa Clara, CA, USA). All steps of this experiment were performed under minimal light conditions. Images were captured using an A1/Ni-E microscope (Nikon, Tokyo, Japan), and image analysis was performed using ImageJ software.

Live imaging of A$\beta$ clearance by BV2 microglia was observed under similar conditions. The BV2 microglia were incubated in confocal dishes, treated with 200 ng/mL LPS or PBS for 30 minutes, and then treated with 1 $\mu$M entrectinib or 1% DMSO (vehicle) for 23.5 hours before A$\beta$ treatment. Thereafter, the BV2 microglia were exposed to pre-incubated A$\beta_{1-42}$ for 1 hour immediately before examination by confocal microscopy. Live images were captured with a Dragonfly 502w high-speed confocal microscope system (Andor Technology, Befast, UK) equipped with a $CO_2$ and temperature-controlled chamber (5% $CO_2$ and 37°C). Video was recorded and snapshots were captured using Imaris analysis software.

## 11. Y-maze

The efficacy of entrectinib in alleviating LPS-induced short-term and spatial memory impairments in wild-type mice was evaluated using a Y-maze test. A Y-maze consisted of three arms, each having a size of 35 cm $\times$ 7 cm $\times$ 15 cm, arranged at 120° angles. During each test session, individual mice freely explored the maze for 5 minutes. Mouse movements were recorded with an Exovision XT (Noldus, Leesburg, VA, USA) video camera and subsequently counted manually. The alternation ratio was determined using the following Alternation ratio (%) = (number of alternations / number of alternation attempts) $\times$ 100%          Formula:

## 12. Novel object recognition (NOR) test

A novel object recognition (NOR) test was used to evaluate effects of entrectinib on long-term memory and recognition memory. A NOR device consisted of an open box having a size of 40 cm $\times$ 40 cm $\times$ 25 cm. The test was conducted in two steps of training and testing, with a 24-hour interval between the two steps. In the training step, two identical objects were placed inside the box, and one mouse explored the box for 5 minutes. In the subsequent test step, the mouse was placed back in the box, and explored the box for 5 minutes with one familiar object and one novel object. During the test step, the positions of the objects were installed in the same position to control positional bias. The box and the objects were cleaned with 70% ethanol between tests to remove odor signals. The exploration time was automatically recorded by reviewing the video of each session. Exploration behavior was defined as facing the nose of the mouse toward the object. Object preference for the novel object was calculated using the following

Formula:

$$\text{Object preference (\%)} = \{T_{Novel}/(T_{Framiliar}+T_{Novel})\} \times 100\ \%$$

Here, $T_{Novel}$ represented the time taken to explore a novel object, and $T_{Framiliar}$ represented the time taken to explore a familiar object.

## 13. Statistical analysis

Data analysis was performed using GraphPad Prism 9 software (GraphPad Software, San Diego, CA, USA). An unpaired both-side T-test applied with Welch's correction was used to compare the two groups. For multiple comparisons, one-way ANOVA and Tukey's test were used. Differences were considered significantly at $p < 0.05$. The results were expressed as mean $\pm$ SD (*$p < 0.05$, **$p < 0.01$, ***$p < 0.001$, ****$p < 0.0001$).

**[Example 1] Confirmation of brain-related inflammation inhibition effect**

1-1. TRK inhibition activity

Entrectinib was known to antagonize tropomysin receptor kinase (TRK), and it was intended to determine whether entrectinib inhibited TRK in microglia. The incubated primary microglia were treated with 200 ng/ml LPS or PBS for 15 minutes and then treated with 1 μM entrectinib or 1% DMSO for 30 minutes.

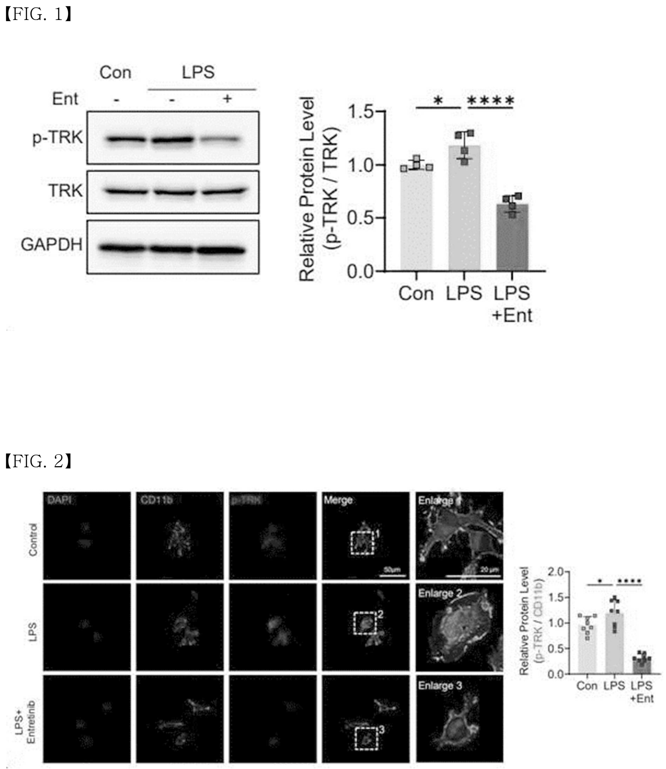

As a result of confirming the protein levels by performing Western blot, it was confirmed that entrectinib significantly inhibited TRK by reducing phosphorylation in LPS-treated primary microglia (FIG. 1). In addition, it was confirmed that surface phosphorylation of TRK was significantly reduced after exposure to entrectinib in LPS-treated primary microglia (FIG. 2).

Therefore, it was confirmed that entrectinib affected the microglial function by antagonizing both TRK and a target receptor.

1-2. Inflammatory response regulation activity

To determine an effect of entrectinib on microglial inflammatory responses, mouse primary microglia were treated with 200 ng/ml LPS or PBS for 30 minutes and then treated with 1 μM entrectinib or 1% DMSO for 5.5 hours.

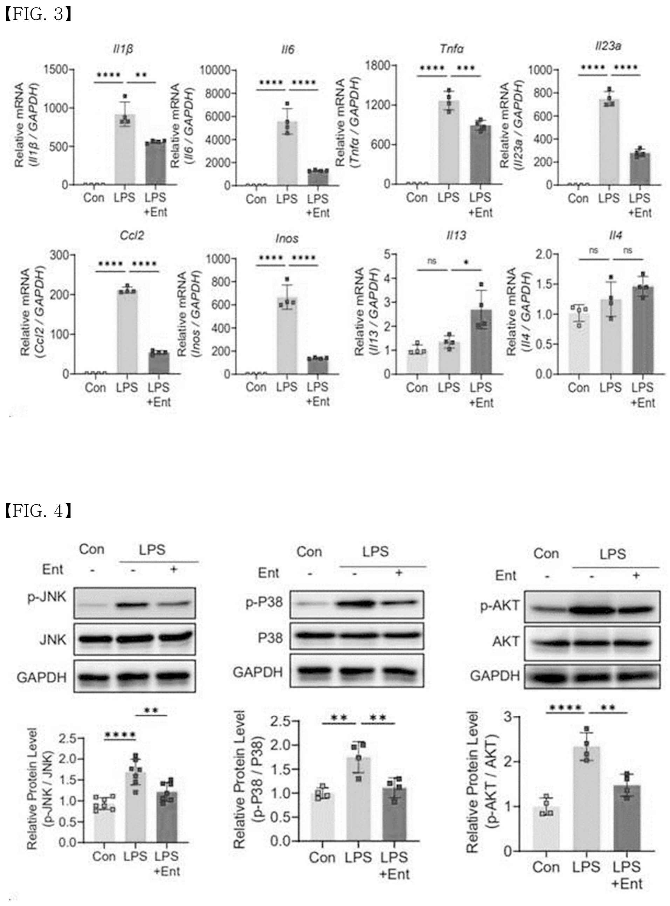

Real-time PCR was performed to determine the gene expression levels of inflammatory cytokines, and as a result, it was shown that entrectinib significantly decreased the increased LPS-induced inflammatory cytokines *Il1β, Il6, Tnfα, and Il23α,* chemokine *Ccl2, and Inos* molecules, and significantly increased an anti-inflammatory cytokine *il13* (FIG. 3).

Therefore, it was confirmed that entrectinib may regulate neuroinflammatory factors that regulated TRK activity in microglia.

1-3. JNK, P38, and AKT phosphorylation regulation activity

Several neuroinflammation-mediated signalings have been identified in microglia, and major inflammatory pathways in the microglia were known as MyD88-MAPK (JNK, P38, ERK, etc.) or PI3K-AKT pathways, which activated pro-inflammatory or anti-inflammatory cytokines or chemokines. To identify signaling molecules involved in the effect of entrectinib on microglial regulation, phosphorylation levels of LPS-induced inflammatory molecules were investigated in microglia after entrectinib treatment.

To confirm the phosphorylation levels, primary microglia of mice were treated with 200 ng/ml LPS or PBS for 15 minutes and then post-treated with 1 μM entrectinib or 1% DMSO for 30 minutes.

Cells were lysed and subjected to Western blotting, and as a result, it was shown that LPS treatment significantly increased p-JNK, p-P38, and p-AKT, and entrectinib significantly reduced these phosphorylation levels (FIG. 4).

Therefore, it was confirmed that entrectinib regulated neuroinflammatory products by reducing p-JNK, p-P38, and p-AKT protein levels in primary microglia.

1-4. Confirmation of regulation of NF-KB and STAT3 signaling pathways

Entrectinib reduced LPS-stimulated inflammation-related signaling molecules, including mitogen-activated protein kinase (MAPK) and AKT, and thus, the association of transcription factors involved in neuroinflammation was investigated.

To confirm this, primary microglia were treated with 200 ng/ml LPS or PBS for 30 minutes and then incubated with 1 μM entrectinib or 1% DMSO for 5.5 hours after LPS or PBS treatment. Nuclear and cytosolic fractions were performed to detect phosphorylation levels.

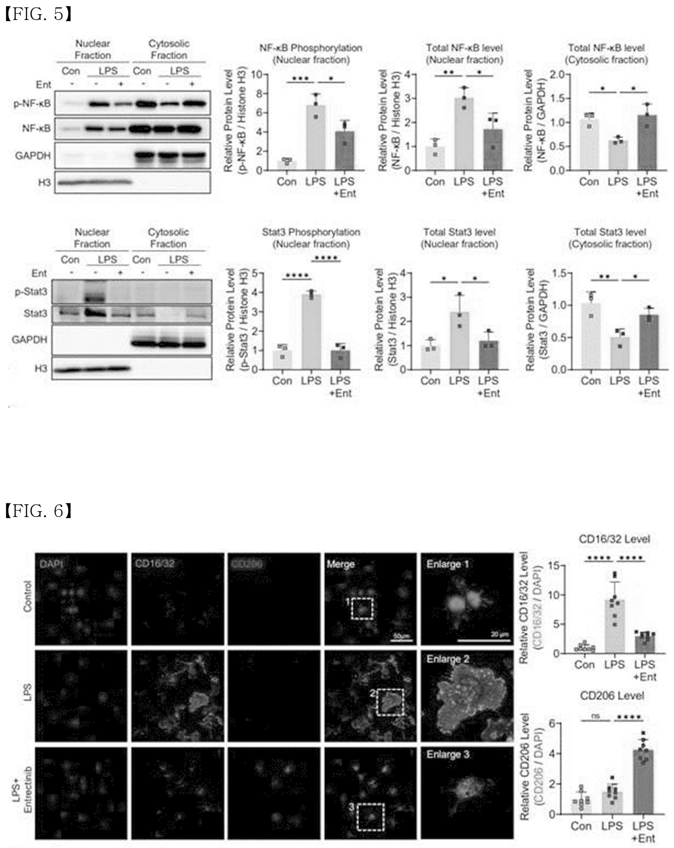

As a result of Western blot analysis, it was shown that entrectinib significantly reduced the increases in LPS-stimulated nuclear p-NF-KB and p-STAT3 levels, but cytosolic phosphorylation levels were increased after entrectinib treatment compared to LPS treatment (FIG. 5). This suggested that LPS stimulated an NF-KB pathway in microglia, translocating p-NF-KB and NF-KB from the cytosol to the nucleus to transcribe genes associated with neuroinflammation.

Therefore, it was confirmed that entrectinib regulated pro-inflammatory and anti-inflammatory factors in LPS-induced microglia by downregulating a Trk-linked NF-κB/STAT3 pathway in primary microglia.

1-5. Confirmation of regulation of CD16/32 and CD206 expression

Entrectinib downregulated the neuroinflammatory response of microglia by inhibiting MAPK, AKT-related p-NF-KB and p-STAT3 levels. In the previous studies, various stages of microglia were developed, but each stage of the microglia played a role in regulating inflammation-related homeostasis in the brain. Among various phenotypes, the systems and effects for restoring the pro-inflammatory related active states and anti-inflammatory response states were well developed. Therefore, whether entrectinib regulated microglial characteristics under LPS conditions may be an important clue to clarify its effects.

To confirm this, the primary microglia were treated with 200 ng/ml LPS or PBS for 30 minutes and then exposed to 1 $\mu$M entrectinib or 1% DMSO for 23.5 hours. Immunofluorescence assay was performed using CD16/32 and CD206, as reactive and anti-inflammatory microglia markers.

As a result, after exposure to entrectinib, it was shown that LPS-stimulated CD16/32 fluorescence intensity was significantly decreased, and CD206 fluorescence intensity was significantly increased after entrectinib treatment compared to LPS treatment (FIG. 6).

Therefore, it was confirmed that entrectinib regulated microgliosis by decreasing CD16/32 in primary microglia, but increasing CD206-associated microglial states.

**[Example 2] Confirmation of effect of increasing phagocytosis**

Since entrectinib had the potential to regulate microglial phenotypes by regulating TRK-associated inflammatory pathways, the effects of entrectinib on microglial phagocytosis were investigated.

2-1. Amyloid beta (A$\beta$) removal activity

The effect of entrectinib on microglial phagocytosis under LPS-induced neuroinflammation conditions was investigated using BV2 microglia.

The BV2 microglia were treated with 200 ng/mL LPS or PBS for 30 minutes, then treated with 1 $\mu$M entrectinib or 1% DMSO for 23.5 hours and treated with A$\beta_{1-42}$ oligomers for 1 hour. Alexa 488 fluorescence was detected in the intracellular membrane of microglia for 1 hour.

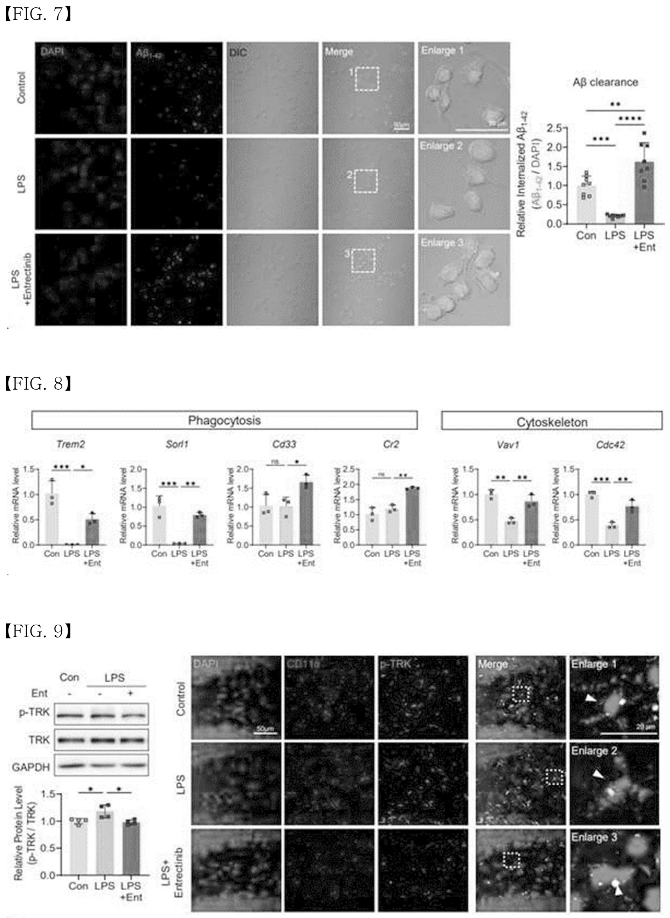

Data from 1 hour after A$\beta_{1-42}$ oligomer treatment showed that entrectinib significantly increased the decrease in LPS-induced A$\beta_{1-42}$ fluorescence intensity, and as a result, it was confirmed that A$\beta_{1-42}$ oligomers were located in the cytosolic region of BV2 microglia (FIG. 7).

Therefore, it was confirmed that entrectinib increased the phagocytosis of microglia for amyloid beta aggregates.

2-2. Phagocytosis regulatory activity

Considering the fact that entrectinib relieved LPS-induced phagocytosis dysfunction in BV2 microglia, association of phagocytosis-related factors was investigated.

To confirm this, the primary microglia were treated with 200 ng/ml LPS or PBS for 30 minutes and then exposed to 1 $\mu$M entrectinib or 1% DMSO for 23.5 hours, and real-time PCR was performed.

As a result, the gene expression of phagocytic receptors such as *Trem2, Sorl1, Cd33, and Cr2* and cytoskeletal factors *Vav1 and Cdc42* was significantly increased after exposure to entrectinib compared to a LPS treatment group (FIG. 8).

Therefore, it was confirmed that entrectinib increased microglial phagocytosis capacity by upregulating the M2 phase of microglia by inhibiting neuroinflammation-related signaling pathways.

**[Example 3] Confirmation of brain-related inflammation inhibition effect through animal experiments**

Since entrectinib downregulated LPS-mediated neuroinflammatory responses *in vitro,* the effect of entrectinib on neuroinflammation *in vivo* was confirmed.

8-week-old male mice were intraperitoneally (i.p.) injected with 10 mg/kg of entrectinib or vehicle (5% DMSO, 10% PEG, 20% Tween 80 in deionized water), and after 30 minutes, administered with LPS (250 ng/kg, i.p.) or PBS daily for 8 days. On the final day, mouse brains were sacrificed, the hippocampi were isolated, and then Western blot and immunofluorescence staining were performed.

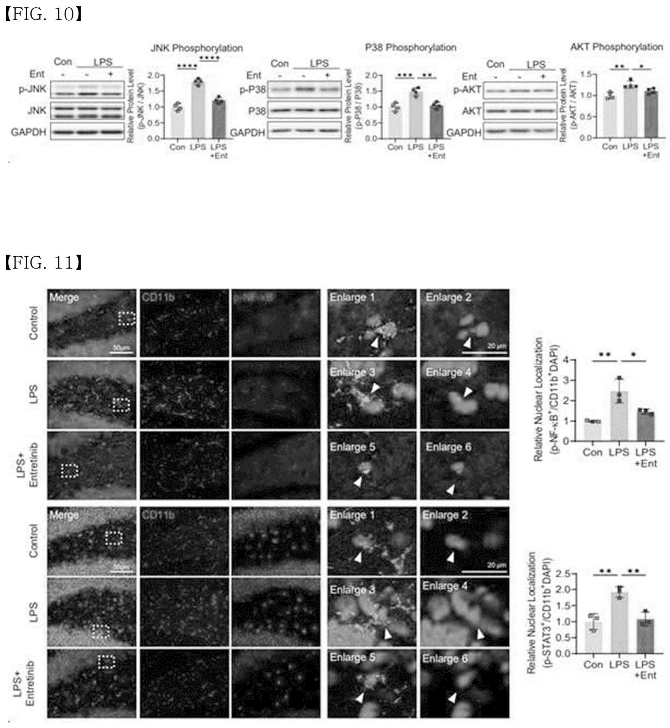

As a result, entrectinib was found to significantly reduce LPS-stimulated TRK phosphorylation in the hippocampi (FIG. 9). Consistently with the *in vitro* results, LPS-induced phosphorylation of JNK, P38, and AKT was significantly downregulated by entrectinib (FIG. 10).

In addition, as the immunofluorescence staining result, it was shown that LPS significantly upregulated p-NF-KB and p-STAT3 levels in the mouse brain hippocampus, but these increases were significantly downregulated by entrectinib treatment (FIG. 11).

Therefore, it was confirmed that entrectinib contributed to neuroinflammatory responses by inactivating the phosphorylation of MAPK/AKT and NF-KB/STAT3 *in vivo.*

**[Example 4] Confirmation of effect of alleviating memory impairment through animal experiments**

Since entrectinib altered LPS-mediated neuroinflammatory responses in the hippocampus, it was intended to determine the effect of entrectinib on neuroinflammation-induced memory impairment in LPS-injected mice.

4-1. Memory impairment alleviation effect

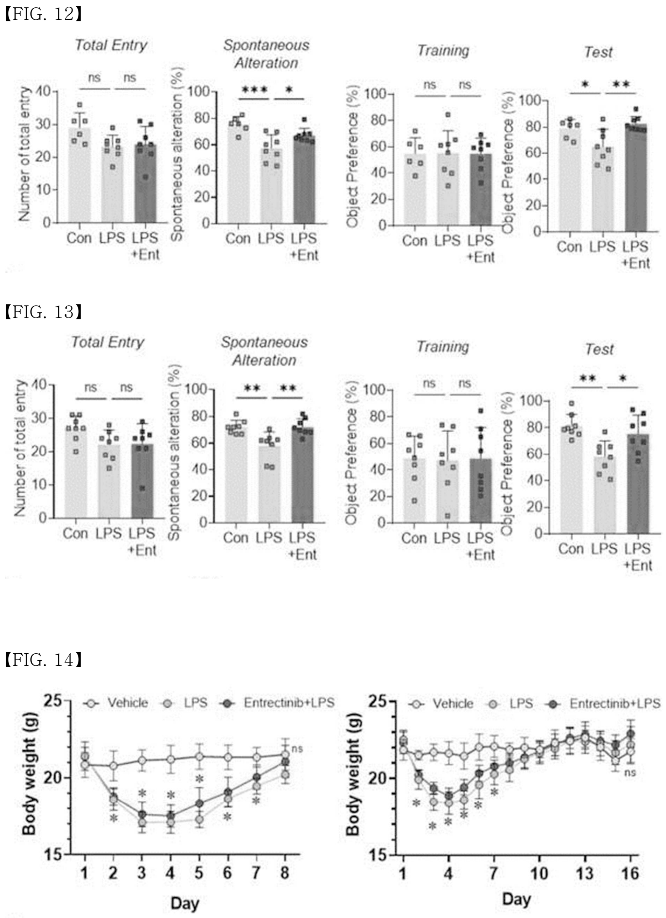

To determine whether entrectinib alleviated memory impairment induced by neuroinflammation after administration, 8-week-old male mice were injected intraperitoneally with LPS (1 mg/kg) or PBS, and then after 30 minutes of injection, administered with 10 mg/kg of entrectinib or PBS daily for 8 or 16 day. Behavioral evaluation using Y-maze and novel object recognition (NOR) tests were conducted on days 7 to 8 or 15 to 16.
As a result, the spontaneous alternation rate (%) and object preference were significantly increased in the Y-maze test and the NOR test, respectively, on days 8 and 16 after entrectinib administration (FIGS. 12 and 13). In addition, it was confirmed that all mice had regained the body weights at the time of behavioral testing (FIG. 14).
Therefore, it was confirmed that entrectinib alleviated memory impairment induced by neuroinflammation in mice.

4-2. Confirmation of synaptic changes

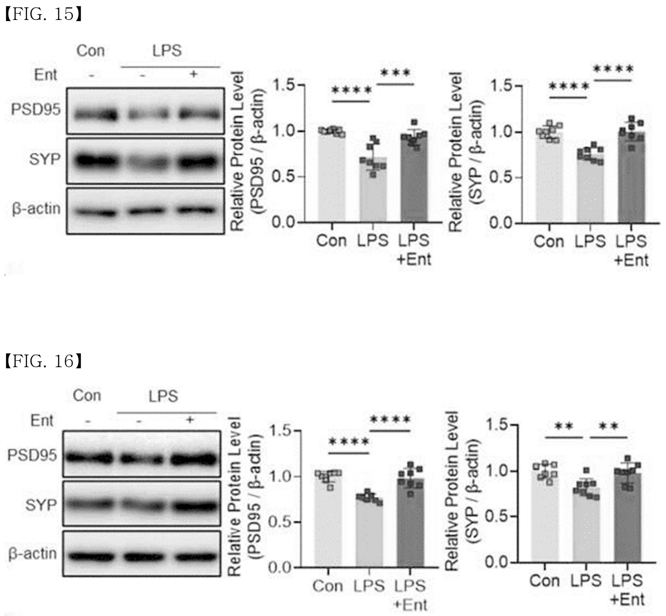

Memory impairment was known to be associated with changes in hippocampal neuron spine formation and synaptic plasticity in the mouse brain. To investigate synaptic changes associated with entrectinib, Western blot analysis was performed using mouse hippocampal tissue after behavioral testing. Synaptophysin (SYP) was used as a representative presynaptic marker, and postsynaptic density protein 95 (PSD95) was used as a postsynaptic marker.
As a result, the PSD95 and SYP levels decreased by LPS exposure were significantly restored after 8 and 16 days of daily administration of entrectinib (FIGS. 15 and 16).
Therefore, it was confirmed that entrectinib contributed to alleviating memory impairment induced by neuroinflammation through upregulation of synaptic proteins in the mouse hippocampus.

**Claims**

1.   A pharmaceutical composition for use in preventing or treating brain-related inflammation disease or neurodegenerative disease comprising entrectinib.

2.   The pharmaceutical composition for use of claim 1, wherein the brain-related inflammation disease or neurodegenerative disease is a patient group with increased tropomysin receptor kinase (TRK) activity.

3.   The pharmaceutical composition for use of claim 1, wherein the composition decreases inflammatory cytokines and increases anti-inflammatory cytokines.

4.   The pharmaceutical composition for use of claim 1, wherein the composition inhibits phosphorylation of JNK, p38, AKT, NF-KB or STAT3.

5.   The pharmaceutical composition for use of claim 1, wherein the composition decreases the expression of CD16/32 and increases the expression of CD206.

6.   The pharmaceutical composition for use of claim 1, wherein the composition reduces amyloid beta (A$\beta$) aggregates.

7.   The pharmaceutical composition for use of claim 1, wherein the composition increases phagocytosis of microglia.

8.   The pharmaceutical composition for use of claim 1, wherein the composition prevents or alleviates memory impairment caused by brain-related inflammation disease or neurodegenerative disease.

9.   The pharmaceutical composition for use of claim 1, wherein the neurodegenerative disease is selected from the group consisting of Alzheimer's disease, Parkinson's disease, Pick's disease, Huntington's disease, dementia, mild cognitive impairment, stroke, and cerebral infarct.

10.   A food composition for use in preventing or alleviating brain-related inflammation disease or neurodegenerative

disease comprising entrectinib.

【FIG. 1】

【FIG. 2】

【FIG. 3】

【FIG. 4】

【FIG. 5】

【FIG. 6】

【FIG. 7】

【FIG. 8】

【FIG. 9】

【FIG. 10】

【FIG. 11】

【FIG. 12】

【FIG. 13】

【FIG. 14】

【FIG. 15】

【FIG. 16】

European Patent Office

Europäisches Patentamt
European Patent Office
Office européen des brevets

# EUROPEAN SEARCH REPORT

Application Number

EP 25 22 4862

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/038415 A1 (CULLGEN SHANGHAI INC [CN]) 27 February 2020 (2020-02-27)<br>* claims 4,124,140 *<br>* page 1, line 10 - line 22 *<br>* page 83, line 31 *<br>* page 42, line 32 - line 33 *<br>* page 34, line 10 - line 13 *<br>* page 1, line 10 - line 12 *<br>----- | 1-10 | INV.<br>A61K31/496<br>A61P25/16<br>A61P25/28 |

**TECHNICAL FIELDS SEARCHED (IPC)**

A61K
A61P

The present search report has been drawn up for all claims

| Place of search | Date of completion of the search | Examiner |
|---|---|---|
| The Hague | 22 April 2026 | Rodrigues Neibecker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 22 4862

22-04-2026

| Patent document cited in search report | Publication date | Patent family member(s) | Publication date |
|---|---|---|---|
| WO 2020038415 A1 | 27-02-2020 | AU 2019323446 A1 | 15-04-2021 |
| | | AU 2025202596 A1 | 01-05-2025 |
| | | BR 112021003098 A2 | 11-05-2021 |
| | | CA 3110267 A1 | 27-02-2020 |
| | | CN 112888681 A | 01-06-2021 |
| | | CN 115626927 A | 20-01-2023 |
| | | CN 118530239 A | 23-08-2024 |
| | | EP 3841098 A1 | 30-06-2021 |
| | | EP 4736882 A2 | 06-05-2026 |
| | | IL 280984 A | 29-04-2021 |
| | | JP 7472103 B2 | 22-04-2024 |
| | | JP 2021535104 A | 16-12-2021 |
| | | KR 20210069634 A | 11-06-2021 |
| | | US 2021363146 A1 | 25-11-2021 |
| | | US 2024400570 A1 | 05-12-2024 |
| | | US 2025388587 A1 | 25-12-2025 |
| | | WO 2020038415 A1 | 27-02-2020 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240197149
- KR 1020250132493
- KR 102718538